# EUROPEAN PATENT APPLICATION

(11) **EP 4 248 961 A1**
(43) Date of publication of application: **27.09.2023**
(21) Application number: 21894778.6
(22) Date of filing: 01.02.2021
(51) Int. Cl.: A61K 31/192, A61K 31/196, A61P 31/14

(54) **ANTIVIRAL COMPOSITION FOR SARS-COV-2 AND HCOV-OC43 COMPRISING RHEIN, MECLOFENAMIC ACID, OR A COMBINATION THEREOF**

(30) Priority: 17.11.2020 KR 20200154110
(71) Applicant: Seoul National University R & DB Foundation, Seoul 08826 (KR); Institute for Basic Science, Yuseong-gu Daejeon 34126 (KR)
(72) Inventor: KIM, V. Narry, Seoul 06292 (KR); KIM, Dongwan, Seoul 08814 (KR); KIM, Hyunjoon, Seoul 07018 (KR)
(74) Representative: J A Kemp LLP
(86) International application number: PCT/KR2021/001312
(87) International publication number: WO 2022/107999

(57) **Abstract**

The present invention relates to an antiviral composition for *Betacoronavirus* comprising rhein and meclofenamic acid as active components. When the antiviral composition of the present invention is used, an antiviral effect is demonstrated whereby the viral RNA level of SARS-CoV-2 and HCoV-OC43 is suppressed, and thus it is possible to treat or prevent COVID-19 or a cold.

## Description

### Technical Field

The present invention was made by Project No. 1711101405 under the support of the Ministry of Science and ICT in Korea and the project was carried out in the project named "Regulatory RNAs in Cell Fate Decision" within the program titled "International Science Business Belt Construction (Support for Research and Operation expenses of the Institute of Basic Science)" by Seoul National University R & DB Foundation under management of the National Research Foundation of Korea, from 03 March 2020 to 28 August 2020.

This application claims priority to and the benefit of Korean Patent Application No. 10-2020-0154110 filed in the Korean Intellectual Property Office on 17 November 2020, the disclosure of which is incorporated herein by reference.

The present invention relates to an antiviral composition for SARS-CoV-2 and HCoV-OC43 of the genus *Betacoronavirus,* the antiviral composition comprising as an active ingredient rhein, meclofenamic acid, or a combination thereof.

### Background Art

Coronavirus (CoV) is an enveloped virus with a positive-sense single-stranded RNA genome and belongs to the family *Coronavirinae.*

The family Coronavirinae is classified into four genera: Alpha-, Beta-, Gamma-, and Delta-coronaviruses. Seven species of coronaviruses that cause infection in humans have been identified so far, of which five species belong to the *Betacoronavirus.* Human coronavirus OC43 (HCoV-OC43) and human coronavirus HKU1 (HCoV-HKU1), which cause mild respiratory symptoms, and severe acute respiratory syndrome coronavirus (SARS-CoV) and Middle East respiratory syndrome coronavirus (MERS-CoV), which cause fatal respiratory infections, belong to *Betacoronavirus.*

Particularly, severe acute respiratory syndrome coronavirus 2 (SARS-CoV-2), which causes coronavirus disease 19 (COVID-19), an acute respiratory disease that began to cause pneumonia of unknown causes in Wuhan, China in December 2019 and became a global pandemic, is also classified into *Betacoronavirus.*

The development of new treatments for COVID-19, an acute respiratory disease caused by SARS-CoV-2 infection, is being actively researched, and drug repurposing or drug repositioning strategy to find preventive and therapeutic effects of COVID-19 in previously developed drugs is being implemented to shorten the time and cost required for new drug development.

For example, remdesivir as an antiviral drug developed as a treatment for Ebola, hydroxychloroquine as an antimalarial drug, lopinavir/ritonavir as an HIV drug, and tocilizumab as an immunosuppressant that suppresses the cytokine storm, and angiotensin-converting enzyme inhibitors (ACE inhibitors) are being administered for emergency use to patients with COVID-19 or are in clinical trials. These potential treatments may relieve the symptoms of COVID-19 or shorten the recovery period thereof, but there is no FDA-approved treatment for COVID-19 until now.

Therefore, the development of antiviral agents for *Betacoronavirus,* which causes epidemic infections in humans, from mild colds to fatal acute respiratory diseases, is urgently needed.

Rhein, an anthraquinone derivative, has been previously used for the treatment of degenerative arthritis due to its anti-inflammatory effect. Meclofenamic acid, an anthranilic acid derivative, has been previously used as a non-steroidal anti-inflammatory drug (NSAID) and an antipyretic. However, the antiviral effects of the two compounds for SARS-CoV-2 and HCoV-OC43 belonging to *Betacoronavirus* have not been known.

### Disclosure of Invention

### Technical Problem

The present inventors have made intensive research efforts to develop an antiviral composition for *Betacoronavirus.* As a result, the present inventors established that a composition comprising as an active ingredient rhein, meclofenamic acid, or a combination thereof significantly inhibited viral RNA, and consequently completed the present invention.

Accordingly, an aspect of the present invention is to provide an antiviral composition, for *Betacoronavirus,* comprising as an active ingredient rhein, meclofenamic acid, or a combination thereof.

Another aspect of the present invention is to provide an antiviral composition for SARS-CoV-2 and HCoV-OC43, the antiviral composition comprising as an active ingredient rhein, meclofenamic acid, or a combination thereof.

Another aspect of the present invention is to provide a pharmaceutical composition for prevention or treatment of coronavirus disease 2019 (COVID-19).

Still another aspect of the present invention is to provide a pharmaceutical composition for prevention or treatment of common cold, acute upper respiratory tract infection, severe acute respiratory syndrome, or viral pneumonia.

### Solution to Problem

In accordance with an aspect of the present invention, there is provided an antiviral composition, for *Betacoronavirus,* comprising as an active ingredient rhein, meclofenamic acid, or a combination thereof.

The present inventors have made intensive research efforts to develop an antiviral composition for *Betacoronavirus.* As a result, the present inventors established that a composition comprising as an active ingredient rhein, meclofenamic acid, or a combination thereof significantly inhibited viral RNA.

The "rhein" of the present invention refers to an anthraquinone derivative, also known as cassic acid and 4,5-dihydroxy-9,10-dioxoanthracene-2-carboxylic acid, which has an anti-inflammatory effect and has thus been used in the existing treatment of degenerative arthritis, wherein the compound is represented by Formula 1.

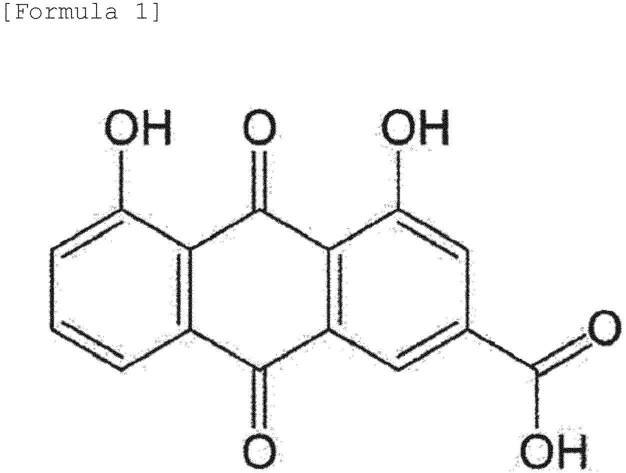

The "meclofenamic acid" of the present invention refers to an anthranilic acid derivative, also known as meclofenamate, which is a cyclooxygenase (COX) inhibitor and has an effect of inhibiting the formation of prostaglandin and has thus been used as an existing non-steroidal anti-inflammatory drug (NSAID) and a fever reducer, wherein the compound is represented by Formula 2.

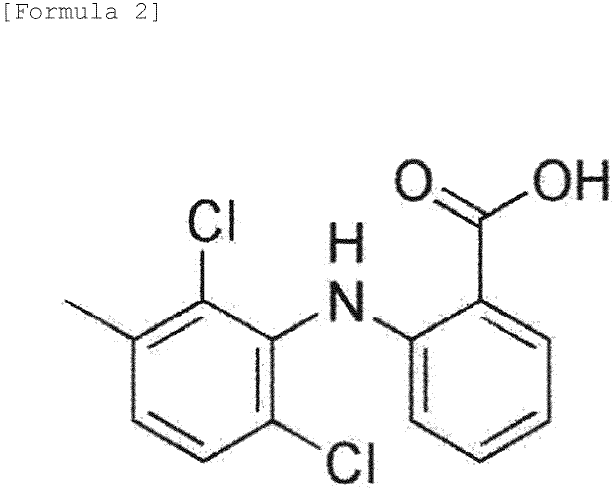

As used herein, the term "comprising as an active ingredient" refers to comprising an amount sufficient to achieve pharmacological efficacy or activity of rhein, meclofenamic acid, or a combination thereof, and means that various ingredients may be additionally added for drug delivery, stabilization, and formulation.

As used herein, the term "antiviral composition" refers to a composition that prevents infection of a virus, inhibits replication of a virus, prevents the spread of a virus, or kills a virus, or a composition for treating a disease caused by viral infection or a symptom expressed by the viral infection, by inhibiting viral entry into host cells, viral genome replication and synthesis, viral genome transcription, viral protein synthesis, reverse transcriptase activity, viral assembly, or viral budding.

As used herein, the term "prevention" refers to a prophylactic or protective treatment of a disease or a disease condition. As used herein, the term "treatment" refers to a reduction, suppression, amelioration, or eradication of a disease condition.

As used herein, the term *"Betacoronavirus"* refers to an RNA virus that infects animals and humans to cause asymptomatic or mild to severe respiratory symptoms, wherein the RNA virus is one of four genera belonging to the family Coronavirinae.

In an embodiment of the present invention, the *Betacoronavirus* of the present invention includes human coronavirus OC43 (HCoV-OC43), human coronavirus HKU1 (HCoV-HKU1), severe acute respiratory syndrome coronavirus (SARS-CoV), Middle East respiratory syndrome coronavirus (MERS-CoV), and severe acute respiratory syndrome coronavirus 2 (SARS-CoV-2).

In an embodiment of the present invention, the *Betacoronavirus* of the present invention includes severe acute respiratory syndrome coronavirus (SARS-CoV) and human coronavirus OC43 (HCoV-OC43).

In an embodiment of the present invention, the active ingredient of the present invention inhibits the viral RNA level of at least one selected from the group consisting of non-structural protein (Nsp), spike (S), envelope (E), membrane (M), nucleocapsid (N), and hemagglutinin esterase (HE) protein of the *Betacoronavirus.*

In an embodiment of the present invention, the active ingredient of the present invention inhibits the viral RNA level of at least one selected from the group consisting of non-structural protein (Nsp), membrane (M) protein, nucleocapsid (N), and hemagglutinin esterase (HE) protein of the *Betacoronavirus.*

As used herein, the term non-structural protein (Nsp) refers to a non-structural protein related to replication and transcription of Coronavirus. There are Nsp1 to Nsp16 in 16 non-structural proteins of Coronavirus, and Nsp3 and Nsp5 have proteolytic activity, and Nsp7, Nsp8, and Nsp12 form a replicase-transcriptase complex (RTC). Nsp12, a key component of RTC, is an RNA-dependent RNA polymerase (RdRp) and directly mediates RNA synthesis in viral replication.

In one embodiment of the present invention, the non-structural protein (Nsp) of the present invention is at least one protein selected from the group consisting of Nsp1, Nsp2, Nsp3, Nsp4, Nsp5, Nsp6, Nsp7, Nsp8, Nsp9, Nsp10, Nsp11, Nsp12, Nsp13, Nsp14, Nsp15, and Nsp16. In another embodiment of the present invention, the non-structural protein of the present invention is Nsp12.

In an embodiment of the present invention, the antiviral composition of the present invention further comprises a pharmaceutically acceptable carrier, vehicle, excipient, stabilizer, or diluent.

The pharmaceutically acceptable carrier, vehicle, excipient, stabilizer, or diluent of the present invention is commonly used in the art to which the present invention pertains, and is a carrier, vehicle, excipient, stabilizer, or diluent that is pharmacologically compatible with the active ingredient of the present invention.

The pharmaceutically acceptable carrier or vehicle of the pharmaceutical composition of the present invention includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, ethanol, dimethyl sulfoxide (DMSO), and the like, but is not limited thereto. The pharmaceutical composition of the present invention may further contain, in addition to the above ingredients, a vehicle, a stabilizer, a diluent, a lubricant, a wetting agent, a sweetening agent, a flavoring agent, an emulsifier, a suspending agent, a preservative, and the like. Suitable pharmaceutically acceptable carriers, vehicles, excipients, stabilizers, or diluents are described in detail in Remington's Pharmaceutical Sciences (19th ed., 1995).

The pharmaceutical composition of the present invention may be orally or parenterally administered, and examples of parenteral administration may include intravenous injection, subcutaneous injection, intramuscular injection, intraperitoneal injection, percutaneous administration, and the like.

An appropriate dose of the pharmaceutical composition of the present invention varies depending on factors, such as a formulating method, a manner of administration, patient's age, body weight, sex, and morbidity, food, a time of administration, a route of administration, an excretion rate, and response sensitivity. The ordinarily skilled practitioner can easily determine and prescribe a dose that is effective for the desired treatment or prevention. The dose of the pharmaceutical composition of the present invention is preferably 0.0001-1000 mg/kg (body weight) per day.

The pharmaceutical composition of the present invention may be formulated using a pharmaceutically acceptable carrier and/or excipient according to a method that could be easily performed by a person having ordinary skills in the art to which the present invention pertains, and the pharmaceutical composition of the present invention may be prepared into a unit dosage form or may be contained in a multi-dose container. The formulation may be in the form of a solution in an oily or aqueous medium, a suspension, or an emulsion, or in the form of an extract, a powder, granules, a tablet, or a capsule, and may further contain a dispersant or a stabilizer.

The mammalian cells used in the present invention includes at least one type of mammalian cells selected from the group consisting of Vero cell line (African green monkey kidney cell line), Calu-3 cell line (human lung adenocarcinoma cell line), HBEC cell line (human bronchial epithelium cell line), and HCT-8 cell line (colon cancer cell line), but is not limited thereto.

The Vero cell line of the present invention is a cell line isolated from kidney epithelial cells of African green monkeys and is commonly used as host cells for virus culture. The Calu-3 cell line of the present invention is a human lung adenocarcinoma cell line and is commonly used in research on respiratory infectious viruses, including SARS-CoV-2 research. The HBEC cell line of the present invention is a human bronchial epithelium cell line and, going one step further in cell culture research, this cell line is a cell line cultured through a cell culture - bronchial epithelium cells differentiated from stem cells - air layer so as to mimic an actual respiratory system. The HCT-8 cell of the present invention is a colon cancer cell line and is a cell line commonly used in HCoV-OC43 research.

In accordance with another aspect of the present invention, there is provided a pharmaceutical composition, comprising the above-described antiviral composition, for prevention or treatment of coronavirus disease 2019 (COVID-19). As used herein, the term "COVID-19" refers to a respiratory disease caused by SARS-CoV-2 infection and indicates coronavirus disease 2019 (COVID-19), showing asymptomatic or mild to severe respiratory symptoms including fever, malaise, cough, dyspnea, pneumonia, phlegm, sore throat, headache, hemoptysis, nausea, diarrhea, and the like. As for the treatment of COVID-19, conservative treatments, such as fluid replacement and/or use of antipyretics, are currently employed as symptomatic treatments, and there is no antiviral drug for SARS-CoV-2.

Since the pharmaceutical composition for prevention or treatment of coronavirus disease 2019 (COVID-19) of the present invention includes the antiviral composition according to another aspect of the present invention described above, the description of overlapping contents therebetween is omitted to avoid excessive complexity herein.

In accordance with still another aspect of the present invention, there is provided a pharmaceutical composition for prevention or treatment of common cold, acute upper respiratory tract infection, severe acute respiratory syndrome, or viral pneumonia.

Since the pharmaceutical composition for prevention or treatment of common cold, acute upper respiratory tract infection, severe acute respiratory syndrome, or viral pneumonia of the present invention includes the antiviral composition according to another aspect of the present invention described above, the description of overlapping contents therebetween is omitted to avoid excessive complexity herein.

In accordance with still another aspect of the present invention, there is provided an antiviral method including administering to a subject in need thereof the above-described antiviral composition, for *Betacoronavirus,* comprising rhein, meclofenamic acid, or a combination thereof as an active ingredient according to another aspect of the present invention.

In accordance with still another aspect of the present invention, there is provided a method for treating a disease caused by viral infection, the method including administering to a subject in need thereof the above-described antiviral composition, for *Betacoronavirus,* comprising as an active ingredient rhein, meclofenamic acid, or a combination thereof according to another aspect of the present invention.

Viruses and diseases, which are targets of the antiviral method and the method for treating a disease, are the same as those defined in the above-described antiviral composition.

In an embodiment of the present invention, the subject of the present invention is a mammal or human. The mammal includes dogs, cats, cows, horses, pigs, mice, rats, chimpanzees, orangutans, baboons, and the like, but is not limited thereto.

Since the above-described antiviral method and method for treating a disease of the present invention are implemented by administering the antiviral composition according to another aspect of the present invention, the description of overlapping contents therebetween is omitted to avoid excessive complexity herein.

### Advantageous Effects of Invention

Features and advantages of the present disclosure are summarized as follows.
(a) The present invention provides an antiviral composition, for *Betacoronavirus,* comprising as an active ingredient rhein, meclofenamic acid, or a combination thereof.
(b) The present invention provides an antiviral composition for SARS-CoV-2 and HCoV-OC43, the antiviral composition comprising as an active ingredient rhein, meclofenamic acid, or a combination thereof.
(c) The present invention provides an antiviral method including administering to a subject in need thereof an antiviral composition, for *Betacoronavirus,* comprising as an active ingredient rhein, meclofenamic acid, or a combination thereof.
(d) The present invention provides a method for treating a disease caused by viral infection, the method including administering to a subject in need thereof an antiviral composition, for *Betacoronavirus,* comprising as an active ingredient rhein, meclofenamic acid, or a combination thereof.
(e) The use of the antiviral composition of the present invention inhibits the RNA level of *Betacoronavirus* and thus can prevent or treat coronavirus disease 2019 (COVID-19), common cold, acute upper respiratory tract infection, severe acute respiratory syndrome or viral pneumonia.

### Brief Description of Drawings

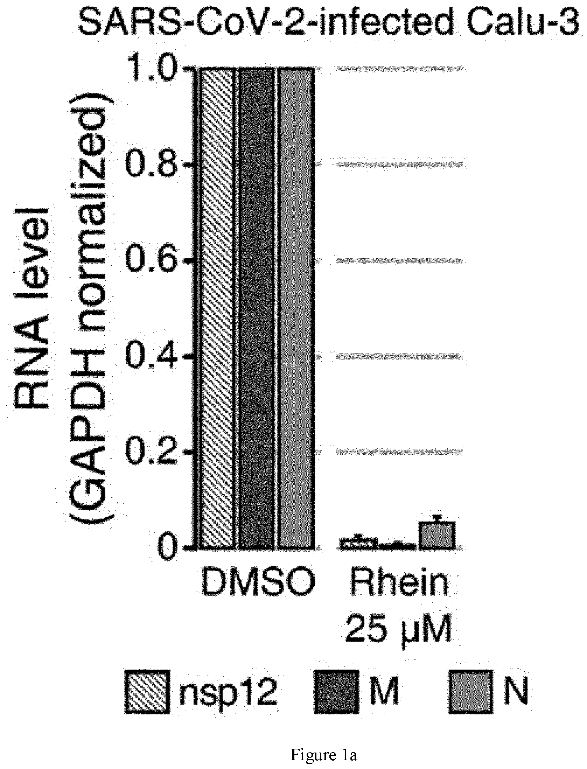

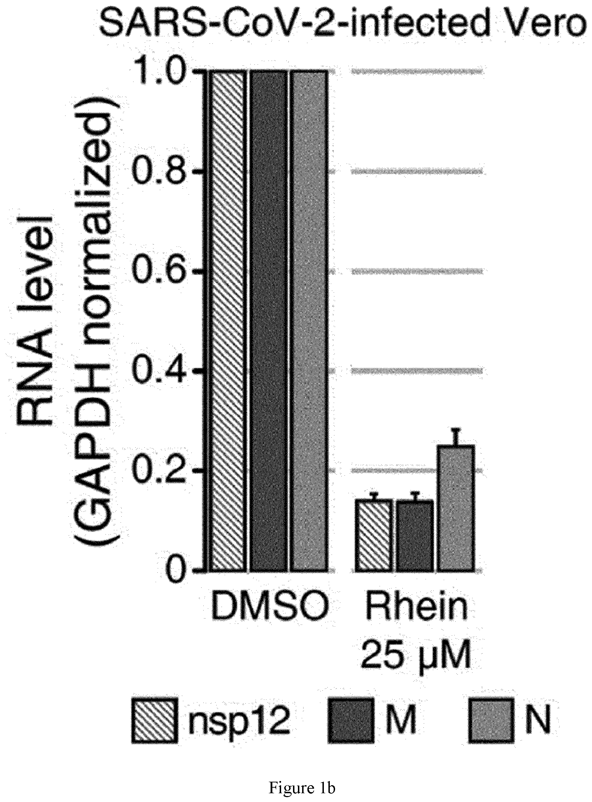

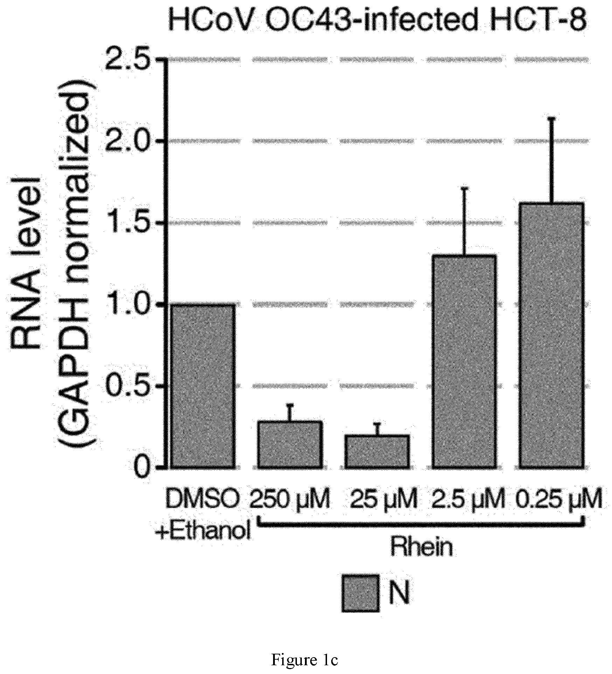

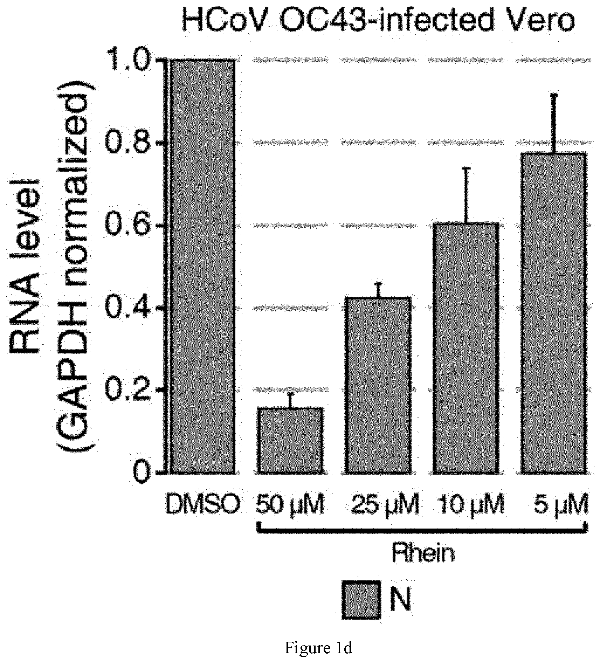

FIGS. 1A to 1D show the viral RNA levels measured by qRT-PCR depending on the treatment of SARS-CoV-2- or HCoV-OC43-infected cells with an antiviral composition comprising rhein. FIG. 1A shows the viral RNA level in SARS-CoV-2-infected Calu-3 cells; FIG. 1B shows the viral RNA level in SARS-CoV-2-infected Vero cells; FIG. 1C shows the viral RNA level in HCoV OC43-infected HCT-8 cells; and FIG. 1D shows the viral RNA level in HCoV OC43-infected Vero cells. All RNA levels were normalized to RNA levels of respective GAPDH.

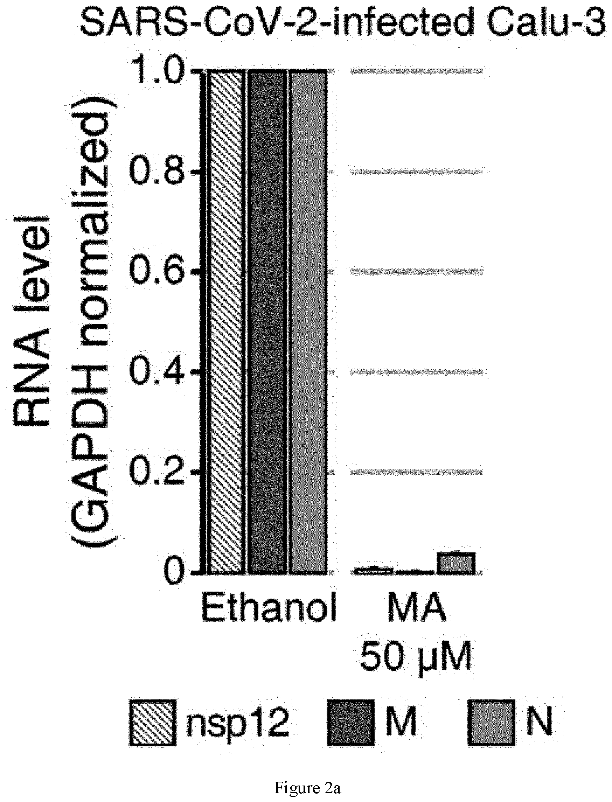

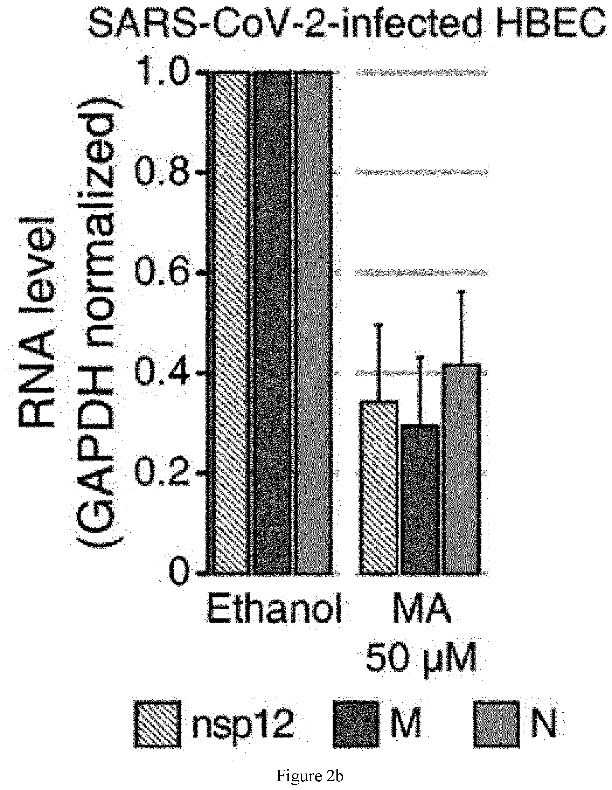

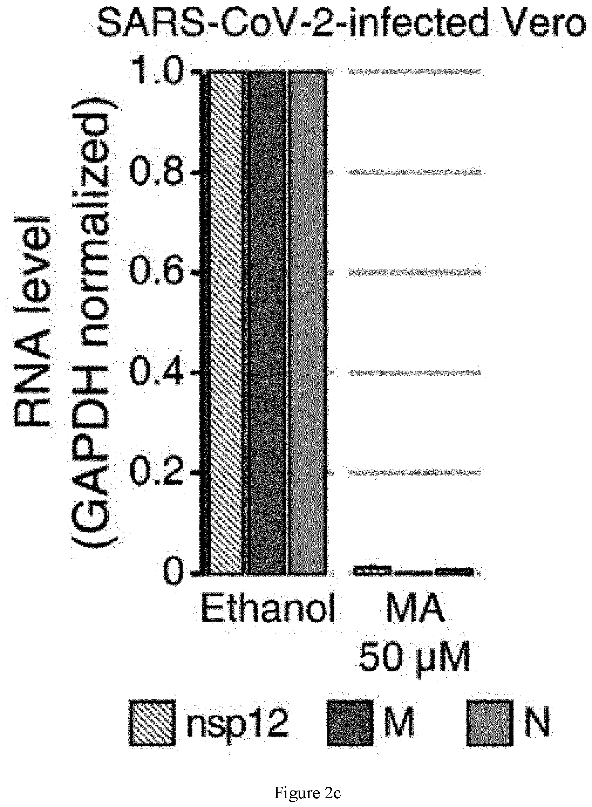

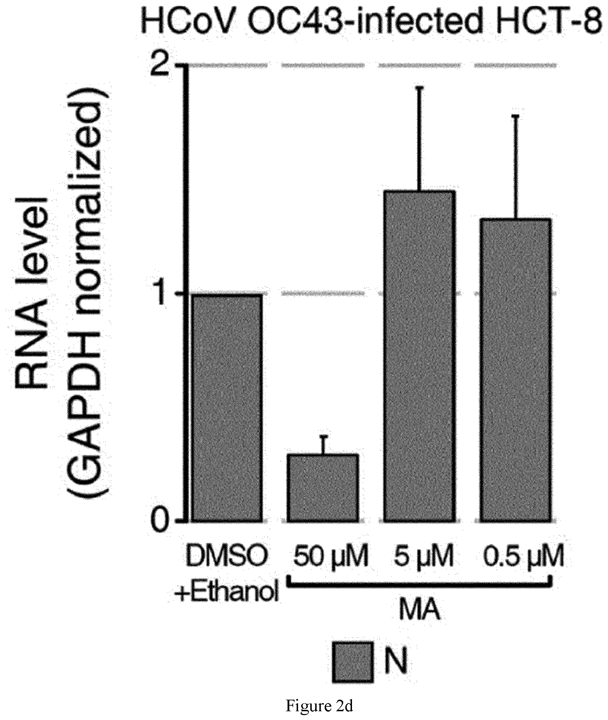

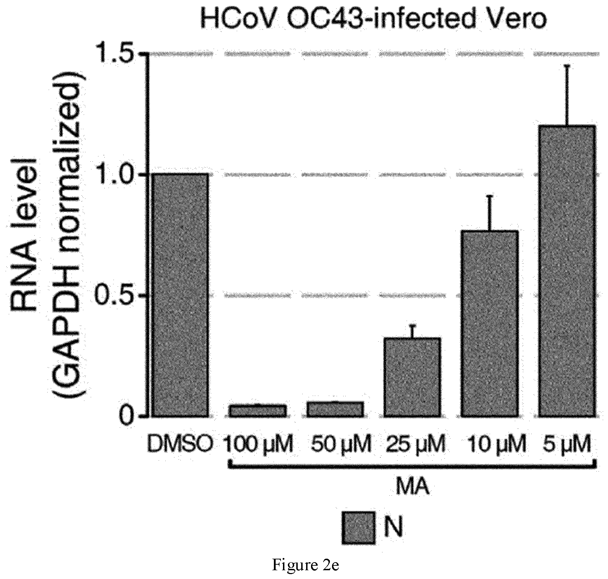

FIGS. 2A to 2E show the viral RNA levels measured by qRT-PCR depending on the treatment of SARS-CoV-2- or HCoV-OC43-infected cells with an antiviral composition comprising meclofenamic acid (MA). FIG. 2A shows the viral RNA level in SARS-CoV-2-infected Calu-3 cells; FIG. 2B shows the viral RNA level in SARS-CoV-2-infected HBEC cells; FIG. 2C shows the viral RNA level in SARS-CoV-2-infected Vero cells; FIG. 2D shows the viral RNA level in HCoV OC43-infected HCT-8 cells; and FIG. 2E shows the viral RNA level in HCoV OC43-infected Vero cells. All RNA levels were normalized to RNA levels of respective GAPDH.

### Best Mode for Carrying out the Invention

Hereinafter, the present invention will be described in more detail with reference to exemplary embodiments. These exemplary embodiments are provided only for the purpose of illustrating the present invention in more detail, and therefore, according to the purpose of the present invention, it would be apparent to a person skilled in the art that these exemplary embodiments are not construed to limit the scope of the present invention.

### Examples

### Example 1: Preparation of antiviral compositions

### Example 1-1: Preparation of antiviral compositions comprising rhein

Rhein was purchased from Sigma-Aldrich (catalogue number R7269) to prepare antiviral compositions comprising rhein at concentrations of 5 µM, 10 µM, 25 µM and 50 µM in the vehicle DMSO. As a control, a composition comprising only DMSO or a mixed solution of 50% (v/v) DMSO and 50% (v/v) ethanol (DMSO + Ethanol) was prepared.

### Example 1-2: Preparation of antiviral compositions comprising meclofenamic acid (MA)

Meclofenamic acid (MA) was purchased from Sigma-Aldrich (catalogue number M4531) to prepare antiviral compositions comprising Meclofenamic acid (MA) at concentrations of 0.5 µM, 5 µM, 10 µM, 25 µM, 50 µM, and 100 µM in the vehicle ethanol. As a control, a composition comprising only ethanol or a mixed solution of 50% (v/v) DMSO and 50% (v/v) ethanol (DMSO + Ethanol) was prepared.

### Example 2: Cell lines

### Example 2-1: Vero cell line

Vero cell line (African green monkey kidney cell line, American Type Culture Collection (ATCC), catalog number: CCL-81) was cultured in Dulbecco's Modified Eagle Medium (DMEM) supplemented with 10% fetal bovine serum (FBS) in an incubator at 37°C and 5% CO₂.

### Example 2-2: Calu-3 cell line

Calu-3 cell line (human lung adenocarcinoma cell line, Korean Cell Line Bank (KCLB), KCLB number: 30055) was cultured in DMEM supplemented with 10% FBS in an incubator at 37°C and 5% CO₂.

### Example 2-3: HCT-8 cell line

HCT-8 cell line (colon cancer cell line, Korean Cell Line Bank (KCLB), KCLB number: 10244) was cultured in Rosewell Park Memorial Institute (RPMI) medium supplemented with 10% FBS in an incubator at 37°C and 5% CO₂.

### Example 2-4: HBEC cell line

HBEC cell line (human bronchial epithelium cell line, ATCC, catalog number: PCS-300-010) was cultured by the protocol provided by STEMCELL^{™} Technologies (*mutatis mutandis*).

More specifically, i) from the start of culture to day 3, HBEC cells were cultured in the culture medium PneumaCult^{™}-Ex plus medium (STEMCELL^{™} Technologies, catalog #05040) added with 0.1 ug/ml hydrocortisone (Sigma, catalog #H0888).

For the differentiation of HBEC cells, ii) from day 4 to day 45 after culture, the HBEC cells cultured for 3 days above were cultured at 3×10⁵ cells/well in a 12-well plate (Transwell^{®} insert, with 0.4 µm-pore membrane between a basal chamber below and an apical chamber above, STEMCELL^{™} Technologies, catalog #05001) while the cells were cultured in 0.5 ml of PneumaCult^{™}-Ex plus medium (STEMCELL^{™} Technologies) in the Apical Chamber. To the basal chamber of the 12-well plate, 1 ml of the culture medium (PneumaCult^{™}-Ex plus medium, STEMCELL^{™} Technologies) per well was added. When the cell confluency reached 100% in the Apical Chamber of the 12-well plate in which HBEC cells were cultured, the culture medium was removed from the Apical Chamber to induce differentiation. Through the differentiation culture, the HBEC cells formed pseudostratified epithelium that is morphologically and functionally similar to human airway epithelium in vivo.

Until 30 days after starting the differentiation culture, the culture medium exchange was performed one time every two days using PneumaCult^{™}-ALI maintenance medium (STEMCELL^{™} Technologies, catalog #05002, #05003, and #05006, 1 µg/ml of hydrocortisone was added) in the basal chamber of the 12-well plate. For the next 7 days, the culture medium exchange was performed twice a week, and for the next 7 days, the culture medium exchange was performed once a week.

iii) On day 45 after culture, the differentiated and maintained HBEC cells were infected with *Betacoronavirus,* and iv) on day 47 after culture, that is, samples for RNA extraction were obtained 48 hours after virus infection.

### Example 3: Viruses

### Example 3-1: SARS-CoV-2

Severe acute respiratory syndrome coronavirus 2 (SARS-CoV-2), which is a pathogen resource distributed by the National Culture Collection for Pathogens under the Korea Disease Control and Prevention Agency, was managed under strict control in biosafety level 3 laboratories at the International Vaccine Institute.

### Example 3-2: HCoV-OC43

Human coronavirus OC43 (HCoV OC43) was obtained from the Korea Bank for Pathogenic Viruses and managed under biosafety level 2 laboratories.

### Example 4: Infection with Betacoronavirus and treatment with antiviral composition

### Example 4-1: Treatment with antiviral composition comprising Rhein in SARS-CoV-2- or HCoV-OC43-infected cell line

### 1) SARS-CoV-2-infected Calu-3 cells

Calu-3 cell line was cultured at 1.75×10⁵ cells/well in three wells of a 12-well plates. After 18 hours of culture, each well was washed with serum-free DMEM to infect each well with SARS-CoV-2 at a multiplicity of infection (MOI) of 0.05 in biosafety level 3 laboratories and cultured in DMEM comprising 2% FBS. Simultaneously with viral infection, the cell culture in each well was treated with an antiviral composition comprising 25 µM rhein and DMSO as a control. Samples for RNA extraction were obtained 24 hours after virus infection.

### 2) SARS-CoV-2-infected Vero cells

Vero cell line was cultured at 1.75×10⁵ cells/well in three wells of a 12-well plate. After 18 hours of culture, each well was washed with serum-free DMEM to infect each well with SARS-CoV-2 at a MOI of 0.05 in biosafety level 3 laboratories and cultured in DMEM comprising 2% FBS. Simultaneously with viral infection, the cell culture in each well was treated with an antiviral composition comprising 25 µM rhein and DMSO as a control. Samples for RNA extraction were obtained 24 hours after virus infection.

### 3) HCoV-OC43-infected HCT-8 cells

HCT-8 cell line was cultured at 2×10⁵ cells/well in two wells of a 12-well plate. After 18 hours of culture, each well was washed with RPMI comprising PBS or 3% FBS, and then each well was infected with HCoV-OC43 in biosafety level 3 laboratories and cultured in RPMI comprising 3% FBS. Simultaneously with viral infection, the cell culture in each well was treated with an antiviral compositions comprising rhein at 0.25 µM, 2.5 µM, 25 µM, and 250 µM and a mixture solution of DMSO and ethanol as a control. After 24 hours of viral infection, each well was washed with RPMI comprising PBS or 3% FBS, and then samples for RNA extraction were obtained.

### 4) HCoV-OC43-infected Vero cells

Vero cell line was cultured at 2×10⁵ cells/well in three wells of a 12-well plate. After 18 hours of culture, each well was washed with DMEM comprising PBS or 3% FBS, and then each well was infected with HCoV-OC43 in biosafety level 3 laboratories and cultured in DMEM comprising 3% FBS. Simultaneously with viral infection, the cell culture in each well was treated with antiviral compositions comprising rhein at 5 µM, 10 µM, 25 µM, and 50 µM and DMSO as a control. After 24 hours of viral infection, each well was washed with DMEM comprising PBS or 3% FBS, and then samples for RNA extraction were obtained.

### Example 4-2: Treatment with antiviral composition comprising meclofenamic acid (MA) in SARS-CoV-2 or HCoV-OC43 infected cell line

Each cell line was infected with each virus by culture under the same or similar conditions as described in Example 4-1 except for the type of antiviral composition for treatment and the HBEC cells infected with SARS-CoV-2, and thus the description of overlapping contents therebetween is omitted to avoid excessive complexity herein.

For SARS-CoV-2-infected Calu-3 cells, SARS-CoV-2-infected HBEC cells, and SARS-CoV-2-infected Vero cells, the cell culture of each well was treated with an antiviral composition comprising meclofenamic acid (MA) at 50 µM and ethanol as a control.

For HCoV-OC43-infected HCT-8 cells, the cell culture of each well was treated with antiviral compositions comprising meclofenamic acid (MA) at 0.5 µM, 5 µM, and 50 µM and a mixture solution of DMSO and ethanol (DMSO + Ethanol) as a control, and for HCoV-OC43-infected Vero cells, the cell culture of each well was treated with antiviral compositions comprising meclofenamic acid (MA) at 5 µM, 10 µM, 25 µM, 50 µM, and 100 µM and DMSO as a control.

### 1) SARS-CoV-2-infected HBEC cells

As in Example 2-4, the cultured HBEC cell line was differentiated and maintained on day 3 after culture, and on day 45 after culture, each well was washed with PBS in biosafety level 3 laboratories, and then infected with SARS-CoV-2 at 3.3 ×10⁴ plaque-forming unit (PFU). Two hours before the viral infection, the cell culture in each well was treated with an antiviral composition comprising MA at 50 µM and DMSO as a control. On day 47 after culture, that is, each well was washed 48 hours after the viral infection, and then samples for RNA extraction were obtained.

### Example 5: RNA isolation and purification

Total RNA was isolated by treating the samples for RNA extraction obtained in Example 4 with Trizol (Invitrogen, catalog number 10296028) as a nucleic acid extraction solution. Total RNA from the SARS-CoV-2-infected cells was purified using the PureLink^{™} RNA Mini Kit (Invitrogen, catalog number: 12183018A) according to the manufacturer's protocol, and total RNA from the HCoV-OC43-infected cells was purified using the RNeasy Mini Kit (Qiagen, catalog number: 74106) according to the manufacturer's protocol. The concentrations of purified RNA were measured using a spectrophotometer (NanoDrop^{™} 2000c Spectrophotometer, ND-2000C, Thermo Scientific).

### Example 6: Quantitative real-time PCR (qRT-PCR)

The purified RNAs obtained from Example 5 were subjected to qRT-PCR by Power SYBR^{™} Green PCR Master Mix (Applied Biosystems^{™}, catalog number: 4367659) and real-time gene amplification equipment (Real-time QuantStudio 3 Real-Time PCR Instrument, Applied Biosystems^{™}).

The sequences of forward (F) and reverse (R) primers used in qRT-PCR experiments are shown in Table 1 below. When the viral RNA levels of non-structural protein 12 (NSP12), membrane (M), and nucleocapsid (N) in the SARS-CoV-2-infected cells, primers having the oligonucleotide sequences shown in SEQ ID NOS: 1 to 6 were used. When the RNA levels of GAPDH in the SARS-CoV-2-infected Calu-3 and HBEC cells, primers having the oligonucleotide sequences shown in SEQ ID NOS: 7 and 8 were used. When the RNA levels of GAPDH in the SARS-CoV-2-infected Vero and HCT-8 cells, primers having the oligonucleotide sequences shown in SEQ ID NOS: 9 and 10 were used. For the measurement of viral RNA levels of nucleotide (N) in the HCoV-OC43-infected cells, primers having the oligonucleotide sequences shown in SEQ ID NOS: 11 and 12 were used.

**Table 1**

| Gene name | F/R | Sequence | SEQ ID NO |
|---|---|---|---|
| NSP12 | F | CAG AGA GCT AGG TGT TGT AC | 1 |
| NSP12 | R | AAG CAC GTA GTG CGT TTA TC | 2 |
| M | F | CGT GCC ACT CCA TGG CAC TAT | 3 |
| M | R | CGT CCT AGA TGG TGT CCA GCA A | 4 |
| N | F | GGC CAG AAG CTG GAC TTC CC | 5 |
| N | R | AGG ATT GCG GGT GCC AAT GT | 6 |
| GAPDH | F | CTC TCT GCT CCT CCT GTT CGA C | 7 |
| GAPDH | R | TGA GCG ATG TGG CTC GGC T | 8 |
| GAPDH | F | TGC CAA CGT GTC AGT GGT G | 9 |
| GAPDH | R | GCC TGC TTC ACC ACC TTC TTG | 10 |
| N | F | TAA GCA ATC CAG TAG TAG AGC G | 11 |
| N | R | TCT AAA CTG GTC GGA CTG AT | 12 |

The cycle threshold (Ct) values of non-structural protein 12 (nsp12), membrane (M) protein, and nucleocapsid (N) protein genes obtained as a result of qRT-PCR experiments were normalized to Ct values of GAPDH as an endogenous control, thereby analyzing the viral RNA levels by using the method of 2^{-ΔΔCt}.

All the experiments of the present invention were repeated at least three times, except for the experiment with HCoV-OC43-infected HCT-8 cells, which was repeated twice. Error bars on the graphs of FIGS. 1A to 1D and 2A to 2E indicate standard errors of the means.

### Example 7: Betacoronaviral RNA inhibitory effect of antiviral composition comprising rhein

### Example 7-1: Viral RNA inhibitory effect of antiviral composition comprising rhein in SARS-CoV-2

In control groups where SARS-CoV-2-infected Calu-3 cells and Vero cells were treated with only DMSO and experimental groups where the cells were treated with an antiviral composition comprising rhein at 25 µM, the viral RNA levels of nsp12, M, and N as measured by qRT-PCR, were normalized to GAPDH and analyzed (FIGS. 1A and 1B). In the SARS-CoV-2-infected Calu-3 cells, the viral RNA levels of nps12, M, and N were all significantly inhibited to less than 5% in the experimental group where the cells were treated with the antiviral composition comprising rhein at 25 µM compared with the control group where the cells were treated with only DMSA (see FIG. 1A). In the SARS-CoV-2-infected Vero cells, the viral RNA levels of nps12, M, and N were all significantly inhibited to about less than 30% in the experimental group where the cells were treated with the antiviral composition comprising rhein (see FIG. 1B).

### Example 7-2: Viral RNA inhibitory effect of antiviral composition comprising rhein in HCoV-OC43

In a control group where HCoV OC43-infected HCT-8 cells were treated with a mixture solution of DMSO and ethanol (DMSO + Ethanol) and experimental groups where the cells were treated with antiviral compositions comprising rhein at 0.25 µM, 2.5 µM, 25 µM, and 250 µM (see FIG. 1C) and a control group where HCoV OC43-infected Vero cells were treated with only DMSO and experimental groups where the cells were treated with antiviral compositions comprising rhein at 5 µM, 10 µM, 25 µM, and 50 µM (see FIG. 1D), the viral RNA levels of N as measured by qRT-PCR were normalized to GAPDH and analyzed.

In the HCoV OC43-infected HCT-8 cells treated with the antiviral compositions comprising rhein at 250 µM and 25 µM and the HCoV OC43-infected Vero cells treated with the antiviral compositions comprising rhein at 5 µM, 10 µM, 25 µM, and 50 µM, the viral RNA levels of N were significantly inhibited. Also, as show0n in FIG. 1D, a dose-dependent effect was confirmed wherein an increasing concentration of rhein in the antiviral composition, used to treat the HCoV OC43-infected Vero cells, increased the inhibition of the RNA level of N.

### Example 8: Viral RNA inhibitory effect of antiviral composition comprising meclofenamic acid (MA)

### Example 8-1: Viral RNA inhibitory effect of antiviral composition comprising meclofenamic acid (MA) in SARS-CoV-2

In control groups where SARS-CoV-2-infected Calu-3 cells, SARS-CoV-2-infected HBEC cells, and SARS-CoV-2-infected Vero cells were treated with only ethanol and experimental groups where the cells were treated with an antiviral composition comprising meclofenamic acid (MA) at 50 µM, the viral RNA levels of nsp12, M, and N as measured by qRT-PCR were normalized to GAPDH and analyzed (see FIGS. 2A, 2B, and 2C). In the SARS-CoV-2-infected Calu-3 cells and the SARS-CoV-2-infected Vero cells, the viral RNA levels of nps12, M, and N were all significantly inhibited to less than 5% in the experimental group where the cells were treated with the antiviral composition comprising MA at 50 µM compared with the control group where the cells were treated with only ethanol (see FIGS. 2A and 2C), and in the SARS-CoV-2-infected HBEC cells, the viral RNA levels were significantly inhibited to about 40% or less (see FIG. 2B).

### Example 8-2: Viral RNA inhibitory effect of antiviral composition comprising meclofenamic acid (MA) in HCoV-OC43

In a control group where HCoV OC43-infected HCT-8 cells were treated with DMSO and ethanol and experimental groups where the cells were treated with antiviral compositions comprising meclofenamic acid (MA) at 0.5 µM, 5 µM, and 50 µM (see FIG. 2D) and a control group where HCoV OC43-infected Vero cells were treated with only DMSO and experimental groups where the cells were treated with antiviral compositions comprising MA at 5 µM, 10 µM, 25 µM, 50 µM, and 100 µM (see FIG. 2E), the viral RNA levels of N as measured by qRT-PCR were normalized to GAPDH and analyzed. In the HCoV OC43-infected HCT-8 cells treated with the antiviral composition comprising MA at 50 µM and the HCoV OC43-infected Vero cells treated with the antiviral compositions comprising MA at 10 µM, 25 µM, 50 µM, and 100 µM, the viral RNA levels of N were significantly inhibited.

## Claims

1. An antiviral composition, for *Betacoronavirus,* comprising as an active ingredient rhein, meclofenamic acid, or a combination thereof.

2. The antiviral composition of claim 1, wherein the *Betacoronavirus* includes human coronavirus OC43 (HCoV-OC43), human coronavirus HKU1 (HCoV-HKU1), severe acute respiratory syndrome coronavirus (SARS-CoV), Middle East respiratory syndrome coronavirus (MERS-CoV), and severe acute respiratory syndrome coronavirus 2 (SARS-CoV2).

3. The antiviral composition of claim 1, wherein the active ingredient inhibits the viral RNA level of at least one selected from the group consisting of non-structural protein (Nsp), spike (S), envelope (E), membrane (M), nucleocapsid (N), and hemagglutinin esterase (HE) protein of the *Betacoronavirus.*

4. The antiviral composition of claim 3, wherein the non-structural protein (Nsp) is at least one protein selected from the group consisting of Nsp1, Nsp2, Nsp3, Nsp4, Nsp5, Nsp6, Nsp7, Nsp8, Nsp9, Nsp10, Nsp11, Nsp12, Nsp13, Nsp14, Nsp15, and Nsp16.

5. The antiviral composition of claim 1, wherein the antiviral composition further comprises a pharmaceutically acceptable carrier, vehicle, excipient, stabilizer, or diluent.

6. A pharmaceutical composition for prevention or treatment of coronavirus disease 2019 (COVID-19), the pharmaceutical composition comprising the antiviral composition of any one of claims 1 to 5.

7. A pharmaceutical composition for prevention or treatment of common cold, acute upper respiratory tract infection, severe acute respiratory syndrome, or viral pneumonia, the pharmaceutical composition comprising the antiviral composition of any one of claims 1 to 5.
